# EUROPEAN PATENT SPECIFICATION

(11) **EP 1 550 649 B1**
(45) Date of publication and mention of the grant of the patent: **11.09.2013**
(21) Application number: 03762940.9
(22) Date of filing: 02.07.2003
(51) Int. Cl.: C07C 209/84

(54) **METHOD FOR PRODUCING RECTIFIED ALKYLDIMETHYLAMINE MIXTURE**
VERFAHREN ZUR HERSTELLUNG EINER REKTIFIZIERTEN ALKYLDIMETHYLAMINMISCHUNG
PROCEDE DE PREPARATION D'UN MELANGE PURIFIE D'ALKYLDIMETHYLAMINES

(30) Priority: 03.07.2002 RU 2002117670
(43) Date of publication of application: 06.07.2005
(73) Proprietor: Nedd Marketing SA, Moscow, 129226 (RU); Mankovich, Leonid, Kiryat-Motskin 26337 (IL)
(72) Inventor: ZOTOV, Vyacheslav Ivanovich, Moscow, 109377 (RU); MANKOVICH, Leonid, 26337 Kiryat-Motskin (IL)
(74) Representative: Ziebig, Marlene
(86) International application number: PCT/RU2003/000287
(87) International publication number: WO 2004/005238

(56) References cited:
- RU-C1- 2 051 897
- US-A- 4 100 209
- US-A- 4 293 716
- US-A- 5 902 903

## Description

### (i) Field of the Invention

The inventive method relates to organic chemistry that includes production of refined alkyldimethylamines, which are the base material for production of quaternary ammonium compounds, and is applicable in production of sanitizers featuring simultaneously detergent properties.

### (ii) Background of the Invention

There is a known method of a two-stage production of alkyldimethylamines from fatty alcohols with the C₆-C₂₀ radicals by hydrochlorination thereof followed by amination in excess methylamine [1] according to the following reactions:

ROH+HCl RCl+H₂O (1)

RCl + 2NH(CH₃)₂ RN(CH₃)₂ + NH(CH₃)₂Cl (2)

Used as a catalyst at the first stage is zinc chloride, and the second stage is amination in excess dimethylamine with the chloroalkanes-to-dimethylamine ratio of 1:5 -20.

In production of tertiary amines, the principal chemical reaction is always accompanied by side reactions, both at the hydrochlorination stage, and at the stage of amination. Therefore, as specified in disclosure (1), production at the amination stage requires a refined mixture of primary chloroalkanes consisting of 50% C₁₂H₂₅Cl and 50% C₁₄H₂₉Cl.

There is a known method (2) for producing alkyldimethylamines of the general formula AlkN(CH₃)₂ from primary fatty alcohols AlkOH by hot hydrochlorination thereof in gaseous hydrogen chloride and subsequent amination of the hydrochlorination product at an elevated temperature and an excessive pressure according to the reactions (1) and (2), where Alk is the hydrocarbon radical C₁₂ - C₂₀. The base primary fatty alcohols individually or in the form of a mixture are subject to hydrochlorination at 140-190° C in the presence of tertiary amines selected from amines with the alkyl radicals C₁₂ -C₂₀ individually or in the form of a mixture in the amount of 6-22% of the mass of batched up alcohols for alcohols with the alkyl radicals C₁₅ - C₂₀ and 8-30% for alcohols with the alkyl radicals C₁₂ - C₁₄, respectively. The amination is carried out at a temperature of 150-155° C and a pressure of 16 atm. Such a solution makes it possible to avoid repeated washing of tertiary amines off zinc chloride.

Production of higher quality alkyldimethylamine requires additional treatment at each stage, as in the patent (2) according to which refined chloroalkanes are aminated by aqueous dimethylamine solution taken in excess, as well as after amination where dimethylamine proper and water must be stripped of dimethylamine salts. The end product also calls for decontamination (clarification) from impurities of other no-purpose alkylamines contaminating the end product.

The known methods of treating tertiary amines are generally based on distillation or vacuum distillation. Thus, the Patent (3) discloses stripping tertiary amines of primary and secondary amines, the Patent (4) discloses separation of an azeotropic mixture of tertiary amines and 2-ethylhexanal from the remaining organic phase, and according to the Patent (3) aqueous tertiary amines solutions are treated by monoaldehyde with subsequent distillation or vacuum distillation. A common disadvantage inherent to said methods is that the end product features a modest purity, and according to the Patents (4) and (5) production of pure tertiary amines requires additional stages of cutting the azeotropic mixture (4) or separating tertiary amines from the resultants of reaction with an additional reagent, monoaldehyde, which, eventually, complicates the process.

The closest prior art of the inventive method is a method disclosed in the Patent (6), according to which a base mixture containing tertiary amines in the liquid phase is cut by vacuum distillation at an elevated temperature. Said method is deficient in that a second stage of stripping, of trimethylamine, is needed, and complete decontamination of the end product from primary and secondary lower amines is impossible.

A common disadvantage inherent to all above methods is that production of a refined mixture of tertiary amines of a narrow fraction consisting of two components of a predetermined composition is impossible.

In case when along with treating a mixture of tertiary amines of a wide spectrum with the alkyl radicals C₆-C₂₅ there is a task of producing a mixture of tertiary amines of a narrow spectrum consisting of two components, e.g., C₁₂ and C₁₄ in a specified proportion, it is necessary to carry out a process with a great many intermediate stages. Separate production of each of the tertiary amines C₁₂ and C₁₄ in a fairly pure form with the view of subsequent mixing thereof presents a formidable challenge requiring numerous process steps to be executed for any one of them individually and, after all, a great power input. Given below is a list of all requisite operations and physico-chemical processes for producing each of them:
- rectification of fatty alcohols;
- production of chloroalkanes;
- treatment of chloroalkanes;
- amination of chloroalkanes with production of alkylamines;
- regeneration of dimethylamine;
- production of tertiary alkylamines;
- attempering of tertiary alkylamines C₁₂ and C₁₄.

### (iii) Disclosure of the Invention

It is a basic object of the invention to produce a refined mixture of alkyldimethylamines consisting essentially of dodecyl dimethylamine and tetradecyl dimethylamine, to cut the number of operations in the treatment process, and, accordingly, to reduce power consumption and cost of the end product, as well as to provide a specified balance of dodecyl dimethylamine and tetradecyl dimethylamine therein.

In a method for producing a mixture of refined alkyldimethylamines with a controllable balance of dodecyl dimethylamine and tetradecyl dimethylamine as claimed in the invention, it is proposed that stripping the base tertiary amines mixture of dimethylamine, alkanes and chloroalkanes, alkyldimethylamines with the alkyl radicals falling beyond the target fraction, and other impurities is combined with the preparation of a specified balance of dodecyl dimethylamine and tetradecyl dimethylamine in the mixture by way of rectification of the base mixture.

This object is accomplished by the organization of the rectification process temperature and refluxing. In pursuing the process parameters of the treatment method, it was unexpectedly found that impurities of dimethylamine, light fractions of alkyl chlorides and alkylamines break away completely and are carried over without condensing in a water-cooled reflux exchanger. At the same time, heavy fractions of impurities accumulate in still bottoms. Thus, two fractions, dodecyl dimethylamine and tetradecyl dimethylamine, dwell on the column trays the ratio of which in the end product is regulated by proportioning the reflux, and, if required, the rectification process is repeated until the desired balance of C₁₂ and C₁₄ is achieved whereby the end product contains tetradecyl dimethylamine and dodecyl dimethylamine and the amount of impurities does not exceed 11 mass percent. Furthermore it contains no heavy fractions of alkyldimethylamines with the alkyl radicals C₁₆ and more.

The technical effect is achieved by that in a method for producing a refined mixture of alkyldimethylamines by distillation of a base mixture of tertiary amines in the liquid phase at an elevated temperature, a mixture of straight-run tertiary amines or a mixture of commercial tertiary amines is used as the base mixture, and distillation of said mixture is carried out by a method of rectification at the top tray temperature from about 80° C to about 130° C, and in the still - from about 130° C to about 310° C. The rectification process can be carried out as at the atmospheric pressure, so at a reduced pressure (from 0.1 to 1 kPa), as well as by ducting from 0 to 100% of the reflux for the column irrigation and tapping the remaining part of the reflux as the end product.

The employment of the mixture of straight-run tertiary amines or the mixture of commercial tertiary amines makes it possible to use readily available and reasonably cheap raw materials, which improves cost effectiveness of the process and reduces costs of the end product.

The temperature range at the top tray from about 80° C to about 130° C is essential for the end product skimming. At a temperature lower than about 80° C, evaporation of said fractions does not occur, as a result of which the amount of impurities in the end product increases. At a temperature higher than about 130° C, the end product, the mixture of dodecyl dimethylamine and tetradecyl dimethylamine, starts evaporating and, as a consequence, its yield declines.

The temperature range in the still from about 130° C to about 310° C is optimal for evaporation of the necessary quantity of the base mixture of tertiary amines. A temperature lower than about 130° C is insufficient for evaporation of the necessary quantity of the base mixture. At such temperatures, evaporation must be carried out in fine vacuum, which complicates mechanization of the process. At temperatures higher than about 310° C, power consumption for the mixture evaporation increases with a trifling change in the yield of the end product. Furthermore, such temperatures result in gumming of the base mixture components, their pyrolysis and thermal destruction.

As stated above, the process can be carried out as at the atmospheric pressure, so under vacuum within the range between 0.1 and 1.0 kPa. This range is optimal for producing a refined mixture of alkyldimethylamines. At a pressure below 0.1 kPa, some extra means are required to create fine vacuum, which complicates the process flow sheet.

The balance of dodecyl dimethylamine and tetradecyl dimethylamine in the resultant mixture is controlled by changing the amount of the reflux supplied for irrigation within the range from 0 to 100%, and the remaining part of the reflux is tapped out as the end product. If approximately 100% of the reflux is fed for the column irrigation, the derived end product features the maximum content of dodecyl dimethylamine and some few tetradecyl dimethylamine with the content of other impurities not exceeding 1%. If 100% of the reflux is tapped out as the end product, on the contrary, the content of tetradecyl dimethylamine is the highest, forasmuch as in this case its dodecyl dimethylamine enrichment does not occur. In this case, the column operates in the distillation mode. Of practical interest is the mode in which part of the reflux is ducted for the column irrigation, and the remaining part is tapped out as the end product. A specific amount of the reflux ducted for the column irrigation, and, accordingly, tapped out in the form of the end product, is determined experimentally depending on the composition of the base mixture of tertiary amines, temperature conditions, number of trays, and so on. The process was carried out with the use of a rectification column with the number of trays not exceeding seven. More trays are inexpedient, since in this case, the column height increases without affecting the end product yield and purity.

### (iv) Preferred Specific Embodiments

In embodiment of a method according to the invention, the use was made of a vacuum-jacketed rectification column. After the installation has been assembled, a vacuum pump was switched on, and the installation was checked for tightness under vacuum within the range of 0.1-1.0 kPa (or 0.75-7.5 mm Hg, respectively). With the airtightness of the entire system ascertained, the still was filled by two thirds with a mixture of commercial tertiary amines, a nebulous liquid of yellow color. Thereupon, water was supplied to the cooling system of a reflux exchanger and a receiver, the vacuum pump was switched on, and, upon attainment of the requisite vacuum, the still heating.

In operation, we monitored and regulated the fluid temperature in the still, at the top tray and, for the avoidance of the still fluid overheating, the heater temperature outside of the still accurate to ±0.5° C, and determined vacuum in the system accurate to ±0.1 kPa. After the still fluid had reached its boiling point, and the top tray temperature had stabilized, we commenced tapping of the rectified mixture of tertiary amines.

In view of the fact that the base mixture components have different length of carbon chains, the tapping was carried out within the temperature range not exceeding 20-30° C. The process was terminated when the volume of the tapped fluid fell off sharply, then the receiver was changed and, in some cases, another, higher boiling fraction, was tapped.

Table 1 presents embodiments of the method according to the invention for different rectification conditions, and the results of analysis of the refined mixture of tertiary amines.

As evidenced by data presented in Table 1, the proposed invention renders it possible to derive a refined mixture of tertiary amines consisting of dodecyl dimethylamine and tetradecyl dimethylamine with the amount of impurities not exceeding 11 mass percent and containing no heavy fractions of alkyldimethylamines with the alkyl radicals C₁₆ and more, which accumulate in still bottoms. The proportion of dodecyl dimethylamine and tetradecyl dimethylamine can be further controlled by changing the amount of the reflux supplied for the column irrigation and that tapped out in the form of the end product, as well as by carrying out additional rectification cycles. However, even a single-distilled base mixture of tertiary amines yields the end product with a purity admissible for further application, e.g., for producing disinfectant, detergent, antibacterial, antiseptic and other alkyldimethyl benzyl ammonium chloride preparations.

The proposed invention also enables production of a refined mixture of alkyldimethylamines by a simple and cost-effective method using procurable and inexpensive raw materials.

**Table**

| No. | Rectification conditions | | | Results of analysis of the refined mixture of tertiary amines | | | |
|---|---|---|---|---|---|---|---|
| | tₛₜᵢₗₗ, °C | t_{top tray}, °C | P, kPa | C₁₂, mass % | C₁₄, mass % | Impurities, mass % | Remarks |
| 1 | 132-163 | 80-98 | 0.1 | 56.3 | 32.7 | 11 | C₁₆ and more |
| | | | | | | | - not found |
| | | | | | | | |
| 2 | 305-315 | 130-135 | 0.1-0.3 | 81.6 | 18 | 0.4 | C₁₆ and more |
| | | | | | | | - not found, 20% of the reflux for irrigation |
| 3 | 132-163 | 80-98 | 0.1 | 99 | 0 | 1 | C₁₆ and more |
| | | | | | | | - not found, 100% of the reflux for irrigation |

### List of Reference

1. RU Patent No. 2108321, cl. C 07 C 211/08, C 07 C 209/08, 10.04.1998
2. RU Patent No. 2051897, cl. C 07 C 211/08, 10.01.1996
3. US Patent No. 5481037, cl. C 07 C 209/84, 209/86, 02.01.1996
4. US Patent No. 5177267, cl. C 07 C 45/82, 05.01.1993
5. US Patent No. 6353138, cl. C 07 C 209/84, 05.03.2002
6. US Patent No. 5902903, cl. C 07 C 209/84, 11.05.1999

## Claims

1. A method for producing refined alkyldimethylamine mixtures consisting of tetradecyl dimethylamine and dodecyl dimethylamine with the amount of impurities not exceeding 11 mass percent and containing no heavy fractions of alkyldimethylamines with the alkyl radicals C₁₆ and more, wherein rectification of a base mixture of tertiary amines is carried out in a vacuum or at the atmospheric pressure, at a temperature at the rectification column top tray from 80° C to 130° C, and a temperature in the still from 130° C to 310° C.

2. A method according to claim 1, **characterized in that** rectification is carried out at a pressure between 0.1 and 1.0 kPa.

3. A method as claimed in any of the foregoing claims 1 and 2, **characterized in that** part of the reflux is ducted for irrigation of the rectification column.

4. A method as claimed in claim 3, **characterized in that** the balance of dodecyl dimethylamine and tetradecyl dimethylamine in the resultant mixture is controlled by changing the amount of the reflux supplied for irrigation within the range from 0 to 100% and the remaining part of the reflux is tapped out as the endproduct.

## Patentansprüche

1. Verfahren zur Herstellung raffinierter Alkyldimethylaminmischungen bestehend aus Tetradecyldimethylamin und Dodecyldimethylamin, wobei die Menge an Verunreinigungen 11 Masseprozent nicht übersteigt und keine schweren Fraktionen von Alkyldimethylaminen mit den Alkylradikalen C₁₆ und mehr enthalten sind, wobei die Rektifikation einer Grundmischung von tertiären Aminen in einem Vakuum oder bei Atmosphärendruck bei einer Temperatur am oberen Boden der Rektifikationssäule von 80 °C bis 130 °C und einer Temperatur im Röhrenverdampfer von 130 °C bis 310 °C durchgeführt wird.

2. Verfahren nach Anspruch 1, **dadurch gekennzeichnet, dass** die Rektifikation bei einem Druck zwischen 0,1 und 1,0 kPa durchgeführt wird.

3. Verfahren nach einem der vorhergehenden Ansprüche 1 und 2, **dadurch gekennzeichnet, dass** ein Teil des Rückflusses mit einer Rohrleitung zur Spülung der Rektifikationssäule ausgestattet ist.

4. Verfahren nach Anspruch 3, **dadurch gekennzeichnet, dass** das Gleichgewicht von Dodecyldimethylamin und Tetradecyldimethylamin in der resultierenden Mischung durch Ändern der Menge des zum Spülen angelieferten Rückflusses innerhalb des Bereichs von 0 bis 100 % reguliert wird und der restliche Teil des Rückflusses als Endprodukt ausgeklopft wird.

## Revendications

1. Procédé de production de mélanges d'alkyldiméthylamines raffinées constitués de tétradécyldiméthylamine et de dodécyldiméthylamine avec une quantité d'impuretés n'excédant pas 11 % en poids et ne contenant pas de fractions lourdes d'alkyldiméthylamines avec les radicaux alkyle en C₁₆ et plus, dans lequel la purification d'un mélange de base d'amines tertiaires est réalisée sous vide ou à pression atmosphérique, à une température au niveau du plateau supérieur de la colonne de purification de 80 °C à 130 °C, et une température dans la colonne de 130 °C à 310 °C.

2. Procédé selon la revendication 1, **caractérisé en ce que** la purification est réalisée à une pression comprise entre 0,1 et 1,0 kPa.

3. Procédé selon l'une quelconque des revendications précédentes 1 et 2, **caractérisé en ce qu'**une partie du reflux est canalisée pour l'irrigation de la colonne de purification.

4. Procédé selon la revendication 3, **caractérisé en ce que** l'équilibre de la dodécyldiméthylamine et de la tétradécyldiméthylamine dans le mélange résultant est contrôlé par la modification de la quantité du reflux fournie pour l'irrigation dans la plage allant de 0 à 100 %, et la partie restante du reflux est prélevée comme étant le produit final.
